# EUROPÄISCHE PATENTSCHRIFT

(11) **EP 1 958 587 B1**
(45) Veröffentlichungstag und Bekanntmachung des Hinweises auf die Patenterteilung: **31.10.2012**
(21) Anmeldenummer: 08001874.0
(22) Anmeldetag: 01.02.2008
(51) Int. Cl.: A61B 19/00, H03K 17/96

(54) **Haltevorrichtung für medizinische Zwecke**
Holder for medicinal purposes
Dispositif de retenue à des fins médicales

(30) Priorität: 13.02.2007 DE 102007006891
(43) Veröffentlichungstag der Anmeldung: 20.08.2008
(73) Patentinhaber: University of Dundee, Dundee DD1 4HN (GB)
(72) Erfinder: Brown, Stuart I., St. Andrews, Fife KY 16 8QX (GB)
(74) Vertreter: Hofmeister, Frank

(56) Entgegenhaltungen:
- EP-A- 0 017 318
- EP-A- 1 152 182
- EP-A- 1 557 134
- WO-A-2007/005976
- US-A1- 2002 177 857

## Beschreibung

Die Erfindung betrifft eine Haltevorrichtung für medizinische Zwecke mit einem Tragarm, an dessen distalem Ende mindestens ein medizinisches Instrument auswechselbar festgelegt ist und mit mindestens einem Gelenk zur Positionierung des Tragarms und/oder des medizinischen Instruments, wobei das mindestens eine Gelenk zwischen einem das Gelenk freigebenden und einem das Gelenk arretierenden Zustand verstellbar ist und wobei das mindestens eine Gelenk zur Betätigung des Gelenks mit einem berührungssensitiven Sensor gekoppelt ist.

Derartige Haltevorrichtungen sind bei der Durchführung chirurgischer Eingriffe häufig erforderlich, um medizinische Instrumente verschiedenster Art, wie beispielsweise Retraktoren, Kameras oder Endoskope; über einen längeren Zeitraum in einer bestimmten Position zu halten. Durch die gelenkige Ausgestaltung der Haltevorrichtungen ist es für den Chirurgen möglich, das in der Instrumentenaufnahme gehaltene medizinische Instrument exakt zu positionieren und die eingestellte Position der Haltevorrichtung durch Arretieren des Gelenks bzw. der Gelenke zu fixieren.

Eine gattungsgemäße Haltevorrichtung ist beispielsweise aus der US 6 587 750 B2 bekannt. Bei dieser bekannten Vorrichtung handelt es sich um eine ferngesteuerten Operationsroboter, dessen Handhabe über einen berührungssensitiven Sensor aktivierbar ist. Nachteilig an dieser bekannten Konstruktion ist, dass es insbesondere bei langen und mehrteiligen Tragarmen zu Positionierungsfehlern im Bereich des medizinischen Instruments kommen kann, wenn die bloße Berührung der Handhabe die Haltevorrichtung bereits in einen verstellbaren Zustand überführt. Eine weitere Haltevorrichtung ist beispielsweise aus der DE 195 26 915 B4 bekannt. Bei dieser bekannten Haltevorrichtung werden die Gelenkteile reibschlüssig durch die Federkraft mindestens eines Federelements relativ zueinender arretiert. Das Lösen der Arretierung erfolgt pneumatisch über im Gelenk angeordnete Lamellen, die beispielsweise mit Druckluft beaufschlagt für eine Trennung des Reibschlusses sorgen. Zwar haben sich derartige Haltevorrichtungen in der Praxis durchaus bewährt, jedoch bilden gerade die über den Reibschluss zusammenzuhaltenden Kontaktflächen Probleme bei der Reinigung, da aufgrund der hohen Kontaktkräfte, die notwendig sind, um einen zuverlässigen Reibschluss zu erzeugen, Kratzer in den Kontaktflächen erzeugt werden können, die ihrerseits Keimzellen für Verunreinigungen bilden können

Aus der US 2002/0177857 A1 ist eine weitere Vorrichtung zum Halten von Operationsgegenständen bekannt. Bei diesem bekannten Stativgestänge ist an einem Adapterstück zwischen dem Endoskop und dem Kamerakopf des Endoskops ein Kontakt-Schalter angeordnet. Die Gelenke zum Verstellen des Tragarms sind über elektromagnetische Bremsen arretierbar und wieder freigebar. Für diesen bekannten Kontakt-Schalter ist es notwendig, dass der Schalter vom Operateur gedrückt wird, um die elektromagnetischen Bremsen zur Freigabe der Gelenke ansprechen zu lassen. Am distalen Ende des Tragarms ist eine um zwei rechtwinklig zueinander angeordnete Drehachsen verschwenkbare Instrumentenaufnahme angeordnet, die nicht der Betätigung durch die elektromagnetischen Bremsen und/oder der Betätigung des Kontakt-Schalters unterliegt, so dass die beiden Drehachsen der Instrumentenaufnahme nicht arretierbar ausgebildet sind.

Davon ausgehend liegt der Erfindung die **Aufgabe** zugrunde, eine Haltevorrichtung für medizinische Zwecke zu schaffen, die bei einfachem Aufbau und einfacher Handhabung eine zuverlässige Positionierbarkeit gewährleistet.

Die **Lösung** dieser Aufgabenstellung ist erfindungsgemäß dadurch gekennzeichnet, dass das am distalen Ende des Tragarms angeordnete medizinische Instrument in einer mit dem berührungssensitiven Sensor gekoppelten, verstellbaren Instrumentenaufnahme gelagert ist, und dass der Sensor mit dem am distalen Ende des Tragarms gelagerten medizinischen Instrument so gekoppelt ist, dass einerseits alle Gelenke des Tragarms sowie der verstellbaren Instrumentenaufnahme automatisch in dem Moment freigegeben werden, wenn der Operateur das zu bedienende medizinische Instrument berührt und andererseits alle Gelenke des Tragarms sowie der verstellbaren Instrumentenaufnahme automatisch in dem Moment wieder arretiert werden, wenn der Operateur das zu bedienende medizinische Instrument loslässt.

Durch die erfindungsgemäße Kopplung des Sensor mit dem am distalen Ende des Tragarms gelagerten medizinischen Instrument, wobei der Sensor so geschaltet ist, dass das mindestens eine Gelenk mittels Betätigung des Sensors in den das Gelenk freigebenden Zustand überführbar ist, ist es für den Operateur auf einfache Art und Weise möglich das Freigeben des Gelenks oder der Gelenke ohne Zuhilfenahme eines Werkzeugs oder sonstige direkte handwerkliche Tätigkeit am Gelenk, wie beispielsweise das Lösen einer Verschraubung, erst dann zu bewirken, wenn er das medizinische Instrument berührt und somit direkten Einfluss auf die Positionierung des medizinischen Instruments ausüben kann.

Die erfindungsgemäße Kopplung des berührungssensitiven Sensors mit dem am distalen Ende des Tragarms gelagerten medizinischen Instruments hat den Vorteil, dass das Gelenk automatisch in dem Moment freigegeben wird, wenn der Operateur das zu bedienende medizinische Instrument berührt. Somit kann der Operateur das Instrument direkt und ohne jegliche weitere Tätigkeit nutzen und in die gewünschte neue Stellung überführen. Sobald der Operateur das medizinische Instrument wieder loslässt, wird der berührungssensitive Sensor wieder deaktiviert und das Gelenk zurück in den das Gelenk arretierenden Zustand überführt, so dass das medizinische Instrument bis zur nächsten Freigabe des Gelenks in der nunmehr eingenommenen Stellung verharrt.

Um die Positionierbarkeit des am distalen Ende des Tragarms gelagerten medizinischen Instrument zu erleichtern, ist das am distalen Ende des Tragarms angeordnete medizinische Instrument in einer verstellbaren Instrumentenaufnahme gelagert, wobei die Instrumentenaufnahme ebenfalls mit dem berührungssensitiven Sensor gekoppelt ist, so dass der Operateur mit dem Ergreifen des medizinischen Instruments direkt die vollständige Freigabe des oder der Gelenke des Tragarms sowie der Instrumentenaufnahme bewirken kann, um direkt und ohne zusätzliche Hilfe unter Ausnutzung aller zur Verfügung stehender Freiheitsgrade das medizinische Instrument in die bestmögliche neue Arbeitsstellung überführen zu können.

Schließlich wird gemäß einer praktischen Ausführungsform der Erfindung vorgeschlagen, dass das der Sensor als kapazitiver Sensor ausgebildet ist. Alternativ ist es selbstverständlich auch möglich, den berührungssensitiven Sensor beispielsweise als induktiven Sensor, temperaturempfindlichen Sensor oder verluststromsensitiven Sensor auszugestalten.

Weitere Merkmale und Vorteile der Erfindung ergeben sich anhand der zugehörigen Zeichnung, in der ein Ausführungsbeispiel einer erfindungsgemäßen Haltevorrichtung für medizinische Zwecke nur beispielhaft schematisch dargestellt ist, ohne die Erfindung auf dieses Ausführungsbeispiel zu beschränken. In der Zeichnung zeigt:
- Fig. 1: eine schematische Darstellung einer erfindungsgemäßen Haltevorrichtung für medizinische Zwecke;

- Fig. 2: einen schematischen elektrischen Schaltplan für einen kapazitiven berührungssensitiven Sensor und
- Fig. 3: eine schematische Darstellung der elektrischen Spannungssignale der Schaltung gemäß Fig. 2.

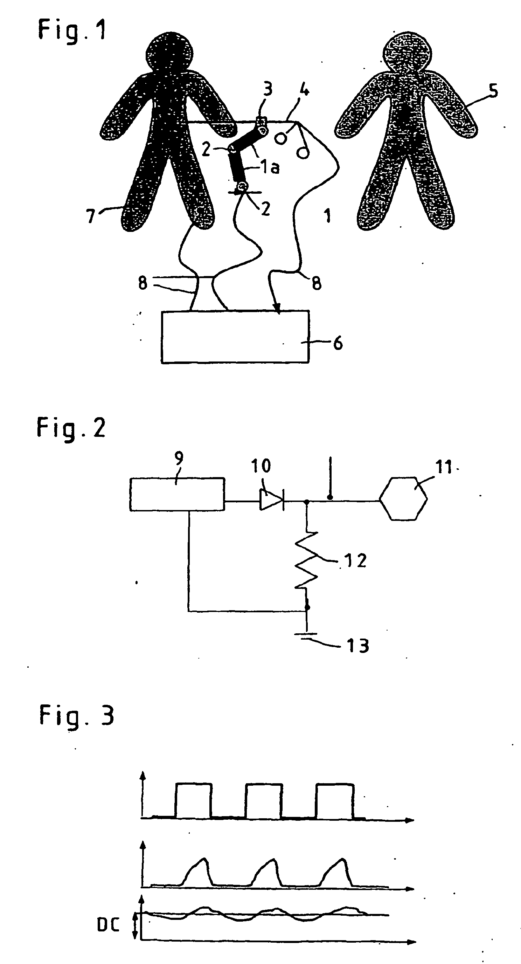

Die Abbildung Fig. 1 zeigt schematisch eine Haltevorrichtung für medizinische Instrumente. Diese Haltevorrichtung besteht im Wesentlichen aus einem aus mehreren Tragarmteilen 1a bestehenden Tragarm 1, wobei die einzelnen Tragarmteile 1a des Tragarms 1 über Gelenke 2 relativ zueinander verschwenkbar miteinander verbunden sind. Im Bereich seines proximalen Endes ist der Tragarm 1 beispielsweise über eine Einspannvorrichtung am Operationstisch festlegbar. Am distalen Ende weist der Tragarm 1 eine Instrumentenaufnahme 3 zur Aufnahme eines über die Haltevorrichtung zu positionierenden medizinischen Instruments 4 auf.

Derartige Haltevorrichtungen sind bei der Durchführung chirurgischer Eingriffe häufig erforderlich, um medizinische Instrumente 4 verschiedenster Art, wie beispielsweise Retraktoren, Kameras oder Endoskope, über einen längeren Zeitraum in einer bestimmten Position zu halten. Durch die gelenkige Ausgestaltung der Haltevorrichtung ist es für den Chirurgen 5 möglich, das medizinische Instrument 4 exakt zu positionieren und die eingestellte Position der Haltevorrichtung durch Arretieren des Gelenks 2 bzw. der Gelenke 2 zu fixieren. Neben der endoskopischen Chirurgie finden derartige Haltevorrichtungen auch in der offenen Chirurgie Anwendung.

Um das in der Instrumentenaufnahme 3 am distalen Ende des Tragarms 1 gelagerte medizinische Instrument 4 in eine neue Arbeitsposition überführen zu können, sind die Gelenke 2 sowie die Instrumentenaufnahme 3 so ausgebildet, dass diese zwischen einem die Bewegung freigebenden und einer arretierten, den Tragarm fixierenden Zustand verstellbar sind. Bei der dargestellten Ausführungsform sind die Gelenke 2 und die Instrumentenaufnahme 3 mit einem berührungssensitiven Sensor 6 gekoppelt, wobei der Sensor 6 im vorliegenden Fall am medizinischen Instrument 4 angeordnet ist.

Der berührungssensitive Sensor 6 ist so ausgelegt, dass er, sobald der Chirurg 5 das medizinische Instrument 4 ergreift und somit auch den Sensor 6 berührt, die Gelenke 2 und die Instrumentenaufnahme 3 in den die Bewegung freigebenden Zustand überführt werden, so dass der Chirurg 5 das medizinische Instrument 4 nunmehr in eine neue Arbeitsposition überführen kann. Sobald der Chirurg 5 das medizinische Instrument 4 wieder loslässt und somit auch den berührungssensitiven Sensor 6 nicht mehr berührt, gehen die Gelenke 2 und die Instrumentenaufnahme 3 wieder in den arretierenden Zustand über, in der das medizinische Instrument 4 in der eingestellten Stellung am Tragarm 1 verharrt.

Bei dem dargestellten Ausführungsbeispiel ist der berührungssensitive Sensor 6 als kapazitiver Sensor ausgelegt, selbstverständlich ist es jedoch auch möglich, den berührungssensitiven Sensor 6 beispielsweise als induktiven Sensor, temperaturempfindlichen Sensor oder verluststromsensitiven Sensor auszugestalten.

Die Arbeitsweise des kapazitiven Sensors 6 beruht auf dem Vergleich eines ungestörten Stromkreises mit bekannter elektrischer Kapazität mit der elektrischen Kapazität des Stromkreises, wenn der Chirurg 5 den Sensor 6 respektive das mit dem Sensor 6 ausgestattete medizinische Instrument 4 berührt.

In Fig. 1 ist schematisch der Aufbau einer mit einem kapazitiven Sensor 6 ausgestatteten Haltevorrichtung für medizinische Instrumente dargestellt.

Das medizinische Instrument 4 ist in der Instrumentenaufnahme 3 am distalen Ende des über Gelenke 2 verstellbaren Tragarms 1 gelagert, wobei die Gelenke 2 und die Instrumentenaufnahme 3 durch Ansteuern einer externen Kraft zwischen einem die Bewegung dieser Bauteile 2 und 3 freigebenden und einer diese Bauteile 2 und 3 arretierenden Zustand verstellbar sind. Der Tragarm 1 selbst ist über sein proximales Ende beispielsweise über eine Einspannvorrichtung am Operationstisch festlegt. Ein zu operierender Patient 7, der Tragarm 1 und das medizinische Instrument 4 sind über Leitungen 8 elektrisch mit dem kapazitätssensitiven Stromkreislauf verbunden. Weiterhin umfasst dieser Stromkreislauf mindestens einen Ausgang, um die Gelenke 2 des Tragarms 1 auf den arretierten oder freigegebenen Zustand hin zu überwachen.

Alternativ zu der dargestellten Ausführungsform kann auf die das medizinische Instrument 4 mit dem kapazitätssensitiven Stromkreislauf verbindende Leitung 8 verzichtet werden, wenn das medizinische Instrument 4 und der Tragarm 1 aus Metal bestehen. In diesem Fall läuft der kapazitive Strom durch das medizinische Instrument 4 und den Tragarm 1, wodurch die Bewegungsfreiheit des medizinischen Instruments 4 deutlich verbessert wird.

Der schematisch in Fig. 2 dargestellte kapazitätssensitive Stromkreislauf der Haltevorrichtung gemäß Fig. 1 ist so ausgelegt, dass die Gelenke 2 des Tragarms 1 sowie die Instrumentenaufnahme 3 im arretierten Zustand verharren, in der das medizinische Instrument 4 eine einmal eingestellte Position beibehält, wenn der Chirurg 5 das medizinische Instrument 4 nicht berührt.

Wenn der Chirurg 5 das medizinische Instrument 4 benutzen und in eine andere Lage verstellen will, ergreift er das medizinische Instrument 4. Dieses Erfassen des medizinischen Instruments 4 durch den Chirurgen 5 erfasst der kapazitätssensitiven Stromkreislauf aufgrund der dem System zugeführten Fremdkapazität. Diese Änderung Kapazitätsänderung bewirkt das Lösen der Gelenke 2 und der Instrumentenaufnahme 3, so dass das medizinische Instrument 4 zur Benutzung freigegeben wird.

Der in Fig. 2 beispielhaft dargestellte Aufbau des kapazitätssensitiven Stromkreislauf des berührungssensitiven Sensors 6 umfasst einen digitalen Oszillator 9, zur Erzeugung eines wellenförmigen Spannungssignals bekannter Frequenz, das zwischen einer niedrigen und einer hohen Spannung schwingt, wie dies in der oberen Grafik der Abbildung Fig. 3 dargestellt ist.

Der Oszillator 9 ist über eine Diode 10 mit der berührungssensitiven Oberfläche 11 verbunden, die eine geringe Eigenkapazität aufweist. Wenn der Sensor 6 nicht berührt wird, wird die geringe Kapazität der berührungssensitiven Oberfläche 11 von dem Signal mit hoher Spannung schnell geladen. Dahingegen wird die Kapazität der berührungssensitiven Oberfläche 11 in der Phase der niedrigen Oszillatorspannung schnell über den Widerstand 12 zur Erdung 13 hin entladen. Unter diesen Bedingungen ist das Ausgangssignal des Sensors 6 eine Welle, die zwischen mit der selben Frequenz wie der Oszillator 9 zwischen hohen und niedrigen Spannungen schwingt, wie dies in der mittleren Grafik in Abbildung Fig. 3 dargestellt ist.

Wenn der Chirurg 5 die berührungssensitive Oberfläche 11 berührt, wird die Kapazität des menschlichen Körpers, die wesentlich größer ist als die Kapazität der berührungssensitiven Oberfläche 11, zu dem Stromkreislauf hinzuaddiert. In der Literatur wird für die Kapazität des menschlichen Körpers ein Wert von etwa 66pF genannt. Der Oszillator 9 lädt diese Kapazität während der Signalphase mit hoher Spannung auf, jedoch ist die Ableitung zur Erdung 13 während der Signalphase mit niedriger Spannung gering, da der Widerstand 12 die Stromableitung begrenzt. Das Ergebnis ist, dass die Spannung der berührungssensitiven Oberfläche 11 niemals wieder auf Null absinkt, solange der Sensor 6 berührt wird. Das Ausgangssignal unter diesen Umständen ist eine schwingende Wellenform mit einem festen Gleichspannungsvorlauf DC, wie dies in der unteren Grafik in Abbildung Fig. 3 dargestellt ist. Der berührte Zustand der berührungssensitiven Oberfläche 11 kann somit vom unberührten Zustand durch das Vorliegen dieses Gleichspannungsvorlaufs DC unterschieden werden.

Um zwischen den beiden wellenförmigen Spannungssignalen des berührten und unberührten Zustands am Ausgang des Sensors 6 unterscheiden zu können, müssen weitere, nicht dargestellte Stromkreisläufe addiert werden. Diese "Unterscheidungskreisläufe" ihrerseits können verwendet werden, um elektromechanische Bauelemente anzusteuern, die den Tragarm 1 und das medizinische Instrument 4 freigeben oder arretieren.

Die Unterscheidung zwischen den beiden Wellenformen kann beispielsweise mittels einer einfachen logischen Schaltung erfolgen. Durch Abgleichen des Wertes des Widerstands 12 sowie der Frequenz des Oszillators 9 und Anpassen des "Unterscheidungskreislaufs" kann die Sensitivität des gesamten Systems eingestellt werden.

Um sicherzustellen, dass die Kapazität des Patienten 7 nicht als Störkapazität in Erscheinung tritt, muss sichergestellt werden, dass der Patient 7 permanent mit dem kapazitätssensitiven Stromkreislauf verbunden ist und somit die Kapazität des Patienten 7 fortwährend zur Kapazität der berührungssensitiven Oberfläche 11 hinzuaddiert wird. Das Einleiten der Patientenkapazität in das System kann beispielsweise über aus der Elektrochirurgie bekannte Erdungspads erfolgen.

Das System wird dann so eingestellt, dass die Kapazitäten der berührungssensitiven Oberfläche 11 und des Patienten 7 über den Widerstand 12 entladen werden können, wenn der Sensor 6 nicht berührt wird und die Oszillatorspannung niedrig ist. Dahingegen wird der Gleichspannungsvorlauf DC der Kapazität des Chirurgen 5 hinzugefügt, sobald der Chirurg 5 den Sensor 6 berührt. Auf diese Weise verbleibt das System sensitiv gegenüber der Berührung durch den Chirurgen 5, jedoch unempfindlich gegenüber der Kapazität des Patienten 7.

### Bezugszeichenliste

- 1: Tragarm
- 1a: Tragarmteil
- 2: Gelenk
- 3: Instrumentenaufnahme
- 4: medizinisches Instrument
- 5: Chirurg
- 6: Sensor
- 7: Patient
- 8: Leitung
- 9: Oszillator
- 10: Diode
- 11: berührungssensitive Oberfläche
- 12: Widerstand
- 13: Erdung

- DC: Gleichspannungsvorlauf

## Patentansprüche

1. Haltevorrichtung für medizinische Zwecke mit einem Tragarm (1), an dessen distalem Ende mindestens ein medizinisches Instrument (4) auswechselbar festgelegt ist und mit mindestens einem Gelenk (2) zur Positionierung des Tragarms (1) und/oder des medizinischen Instruments (4), wobei das mindestens eine Gelenk (2) zwischen einem das Gelenk (2) freigebenden und einem das Gelenk (2) arretierenden Zustand verstellbar ist und wobei das mindestens eine Gelenk (2) zur Betätigung des Gelenks (2) mit einem berührungssensitiven Sensor (6) gekoppelt ist,
**dadurch gekennzeichnet,**
**dass** das am distalen Ende des Tragarms (1) angeordnete medizinische Instrument (4) in einer mit dem berührungssensitiven Sensor (6) gekoppelten, verstellbaren Instrumentenaufnahme (3) gelagert ist, und dass der Sensor (6) mit dem am distalen Ende des Tragarms (1) gelagerten medizinischen Instrument (4) so gekoppelt ist, dass einerseits alle Gelenke (2) des Tragarms (1) sowie der verstellbaren Instrumentenaufnahme (3) automatisch in dem Moment freigegeben werden, wenn der Operateur das zu bedienende medizinische Instrument (4) berührt und andererseits alle Gelenke (2) des Tragarms (1) sowie der verstellbaren Instrumentenaufnahme (3) automatisch in dem Moment wieder arretiert werden, wenn der Operateur das zu bedienende medizinische Instrument (4) loslässt.

2. Haltevorrichtung nach Anspruch 1, **dadurch gekennzeichnet, dass** der Sensor (6) als kapazitiver Sensor ausgebildet ist.

## Claims

1. A holding device for medical purposes with a supporting arm (1), on the distal end of which at least one medical instrument (4) is fixed in a replaceable manner and with at least one joint (2) for positioning the supporting arm (1) and/or the medical instrument (4), wherein the at least one joint (2) can be adjusted between a state releasing the joint (2) and a state locking the joint (2) and wherein the at least one joint (2) for actuating the joint (2) is coupled to a touch-sensitive sensor (6), **characterized in that** the medical instrument (4) arranged at the distal end of the supporting arm (1) is supported in an adjustable instrument seat (3) coupled to the touch-sensitive sensor (6) and that the sensor (6) is coupled to the medical instrument (4) supported at the distal end of the supporting arm (1) such that, on the one hand, all of the joints (2) of the supporting arm (1) as well as of the adjustable instrument seat (3) are automatically released at the moment when the operator touches the medical instrument (4) to be operated and, on the other hand, all of the joints (2) of the supporting arm (1) as well as of the adjustable instrument seat (3) are automatically locked again at the moment when the operator releases the medical instrument (4) to be operated.

2. The holding device according to claim 1, **characterized in that** the sensor (6) is embodied as a capacitive sensor.

## Revendications

1. Dispositif de support de maintien destiné à des fins médicales, comprenant un bras porteur (1) à l'extrémité distale duquel est fixé, de manière interchangeable, au moins un instrument médical (4), et comprenant au moins une articulation (2) pour positionner le bras porteur (1) et/ou l'instrument médical (4), ladite au moins une articulation (2) pouvant être commutée entre un état libérant l'articulation (2) et un état bloquant l'articulation (2), et ladite au moins une articulation (2) étant couplée, pour l'actionnement de l'articulation (2), avec un détecteur (6) sensible au toucher, **caractérisé en ce que** l'instrument médical (4) agencé à l'extrémité distale du bras porteur (1) est monté dans un récepteur d'instrument (3) réglable, couplé au détecteur (6) sensible au toucher, et **en ce que** le détecteur (6) est couplé à l'instrument médical (4) monté à l'extrémité distale du bras porteur (1) de façon telle, que d'une part toutes les articulations (2) du bras porteur (1) ainsi que du récepteur d'instrument (3) réglable soient automatiquement libérées à l'instant où l'opérateur touche l'instrument médical (4) devant être manié, et que d'autre part toutes les articulations (2) du bras porteur (1) ainsi que du récepteur d'instrument (3) réglable soient à nouveau automatiquement bloquées à l'instant où l'opérateur relâche l'instrument médical (4) devant être manié.

2. Dispositif de support de maintien selon la revendication 1, **caractérisé en ce que** le détecteur (6) est réalisé sous forme de détecteur capacitif.
